# FASCICULE DE BREVET EUROPEEN

(11) **EP 3 295 888 B1**
(45) Date de publication et mention de la délivrance du brevet: **26.01.2022**
(21) Numéro de dépôt: 17187856.4
(22) Date de dépôt: 25.08.2017
(51) Int. Cl.: A61B 50/30, A61B 90/98, A61B 90/00

(54) **DISPOSITIF POUR INSTRUMENT CHIRURGICAL, DISPOSANT DE CAPTEURS POUR LA MEMORISATION D'INFORMATIONS**
VORRICHTUNG FÜR CHIRURGISCHES INSTRUMENT, DAS ÜBER SENSOREN ZUR INFORMATIONSSPEICHERUNG VERFÜGT
DEVICE FOR SURGICAL INSTRUMENT, HAVING SENSORS FOR STORING INFORMATION

(30) Priorité: 16.09.2016 FR 1658709
(43) Date de publication de la demande: 21.03.2018
(73) Titulaire: In'Tech Medical, 62180 Rang du Fliers (FR)
(72) Inventeur: KHALIFE, Patrick, 62180 Rang Du Fliers (FR); HASSANE, François, 62155 Merlimont (FR); LEROY, Xavier, 62170 Campigneulles les Petites (FR); PRUVOST, Laurent, 62152 Neufchatel Hardelot (FR)
(74) Mandataire: HGF

(56) Documents cités:
- WO-A1-2016/075418
- US-A1- 2006 214 791
- US-A1- 2007 160 494
- US-A1- 2010 262 139
- US-A1- 2016 249 919

## Description

### DOMAINE DE L'INVENTION

La présente invention est relative au domaine des instruments chirurgicaux. Elle concerne plus précisément un instrument chirurgical comportant des capteurs et des moyens de mémorisation et de transmission d'informations issues de ces capteurs vers un dispositif extérieur, ainsi qu'un procédé d'utilisation d'un tel instrument chirurgical.

### CONTEXTE DE L'INVENTION

Un instrument chirurgical est soumis durant sa vie à de nombreuses sollicitations. Ces sollicitations peuvent être mécaniques ou physiques et liées à leurs utilisations normales. Leurs natures dépendent alors du type d'instruments : déclenchement d'une action mécanique, torsions, impactions, etc. D'autres sollicitations peuvent être accidentelles : chocs, chutes, etc. Enfin, les instruments chirurgicaux sont soumis à des stérilisations après chaque utilisation qui engendrent des sollicitations importantes en température et en pression.

Ces sollicitations diverses engendrent évidemment une usure de l'instrument et impactent sur son état et sur sa durée de vie.

Il n'est pas possible d'estimer l'état d'un instrument en fonction simplement de la durée de service de celui-ci, car l'état dépend d'une part de son utilisation effective et d'autre part du type de sollicitation qu'il subit. En outre, l'examen visuel ne permet pas d'estimer efficacement son état non plus, car certains types d'usure sont masqués : mécanismes internes, modification structurelle des matériaux, etc.

Or, l'utilisation d'un instrument chirurgical au-delà d'un certain niveau d'usure engendre des risques importants pour le patient sur lequel l'intervention chirurgicale a lieu.

Aussi, actuellement, afin de minimiser le risque en deçà d'un seuil considéré comme acceptable, il est prévu de retirer les instruments du lieu d'utilisation pour réétalonnage ou remplacement, selon une périodicité conservatrice, c'est-à-dire établie pour minimiser les risques et sans considérer le degré d'usure réel de l'instrument.

Ces remplacements et/ou réétalonnages ont un coût significatif qui est supporté, selon les cas, par le fabriquant de l'instrument, par son distributeur ou par le secteur hospitalier.

Le but de la présente invention est de minimiser ce coût en permettant aux différents intervenants de disposer d'informations précises associées au degré d'usure d'un instrument chirurgical. Celui-ci peut alors être remplacé ou ré-étalonné uniquement en cas de nécessité.

Le document WO 2016/075418 A1 montre une boîte de stérilisation permettant une traçabilité et le suivi des instruments chirurgicaux dans un environnement médical tel qu'une enceinte hospitalière.

### RESUME DE L'INVENTION

Le but de la présente invention est de fournir un instrument chirurgical palliant les inconvénients précités.

Plus particulièrement, un objet de l'invention est un instrument chirurgical avec un dispositif localisé sur l'instrument, le dispositif à comportant au moins un capteur, un circuit de contrôle et de traitement des signaux issus dudit au moins un capteur, une mémoire pour stocker des informations issues dudit circuit, et une interface radio pour transmettre lesdites informations à un dispositif extérieur, ledit au moins un capteur, ledit circuit, ladite mémoire et ladite interface étant alimentés par une source d'énergie, ledit circuit, ladite mémoire, ladite interface radio et ladite source d'énergie étant logés dans un ou plusieurs boîtiers hermétiques et résistants à la chaleur et à la pression afin de permettre un fonctionnement autonome du dispositif

Suivant des modes de réalisation préférés, l'invention comprend une ou plusieurs des caractéristiques suivantes qui peuvent être utilisées séparément ou en combinaison partielle entre elles ou en combinaison totale entre elles :
- ledit boitier est un surmoulage en matériau polymère ou thermoplastique ;
- lequel ladite interface radio est une étiquette RFID ;
- ledit au moins un capteur comprend un accéléromètre et un contacteur bilame ;
- ladite interface radio est prévue pour recevoir des secondes informations dudit dispositif extérieur pour les stocker dans ladite mémoire ;
- les capteurs sont prévus pour transmettre une interruption audit circuit, lors de la survenance d'un événement prédéfinis, et dans lequel ledit circuit est adapté pour se maintenir dans un état de veille jusqu'à la réception de ladite interruption et pour alors traiter les signaux issus du capteur correspondant à ladite interruption, puis se remettre en état de veille ;
- ledit circuit est prévu pour sortir périodiquement et temporairement de l'état de veille afin de vérifier la consistance du contenu entre ladite mémoire et sa mémoire interne et l'état de ladite source d'énergie ;
- ledit boîtier est constitué d'un matériau résistant à une température d'environ 135°C degrés et à une pression d'environ 3 bars.

Un autre objet de l'invention est un système comportant un dispositif pour instrument chirurgical tel que précédemment défini, et un dispositif extérieur prévu pour permettre la visualisation des informations reçues de ladite interface radio.

Suivant des modes de réalisation préférés, ce système selon l'invention comprend les caractéristiques suivantes qui sont en combinaison totale entre elles :
- le système comprend une caisse de transport muni lui-même d'un dispositif tel que précédemment défini ;
- ladite caisse contient un ensemble d'instruments chirurgicaux contenant un dispositif tel que précédemment défini.

Un autre objet de l'invention concerne un procédé de gestion de l'instrument chirurgical avec le dispositif, selon la revendication 1, comportant une étape préalable de stockage d'informations dans la mémoire du dispositif associé audit instrument, issues d'au moins un des capteurs intégrés dans ledit dispositif, ladite mémoire étant alimentée par la source d'énergie, ladite mémoire et ladite source étant logées dans au moins un boîtier hermétique et résistant à la chaleur et à la pression afin de permettre un fonctionnement autonome dudit dispositif, puis une étape ultérieure de transmission desdites informations stockées dans la mémoire dudit dispositif vers un dispositif extérieur et d'affichage desdites informations sur une interface homme-machine de ce dispositif extérieur.

Encore un autre objet de l'invention est un procédé de gestion d'un ensemble d'instruments chirurgicaux contenus dans une caisse de transport, ledit procédé comportant
- l'utilisation du procédé précédemment décrit pour chacun des instruments dudit ensemble afin que lesdites informations soient transmises à un dispositif extérieur unique, ainsi que
- une étape préalable de stockage d'informations dans une mémoire d'un dispositif associé à ladite caisse, issues d'au moins un capteur intégré dans ledit dispositif, ladite mémoire étant alimentée par une source d'énergie, ladite mémoire et ladite source étant logées dans au moins un boitier hermétique et résistant à la chaleur et à la pression afin de permettre un fonctionnement autonome dudit dispositif, puis une étape ultérieure de transmission desdites informations stockées dans la mémoire dudit dispositif vers ledit dispositif extérieur, ledit dispositif extérieur permettant l'affichage des informations reçues de ladite caisse et desdits instruments chirurgicaux sur une interface homme-machine.

D'autres caractéristiques et avantages de l'invention apparaîtront à la lecture de la description qui suit d'un mode de réalisation préféré de l'invention, donnée à titre d'exemple et en référence aux dessins annexés.

### BREVE DESCRIPTION DES DESSINS

La figure 1 représente schématiquement un exemple d'architecture fonctionnelle selon un mode de réalisation de l'invention.
La figure 2 représente schématiquement un exemple de procédé pouvant être mis en oeuvre par le circuit de contrôle et de traitement mis en oeuvre dans un mode de réalisation de l'invention.
Les figures 3 et 4 représentent schématiquement deux vues d'un exemple de poignée d'un instrument chirurgical selon un mode de réalisation de l'invention.

### DESCRIPTION DETAILLEE DE L'INVENTION

L'invention est susceptible de s'appliquer à différents types d'instruments chirurgicaux. Pour chaque type d'instruments, des capteurs différents pourront être mis en place afin de fournir des informations qui soient adaptées et pertinentes par rapport au type d'usure ou de sollicitation que peut subir l'instrument ou de suivre des caractéristiques spécifiques de l'instrument, ainsi que permettant aux intervenants de juger de la nécessité de remplacer ou de ré-étalonner l'instrument.

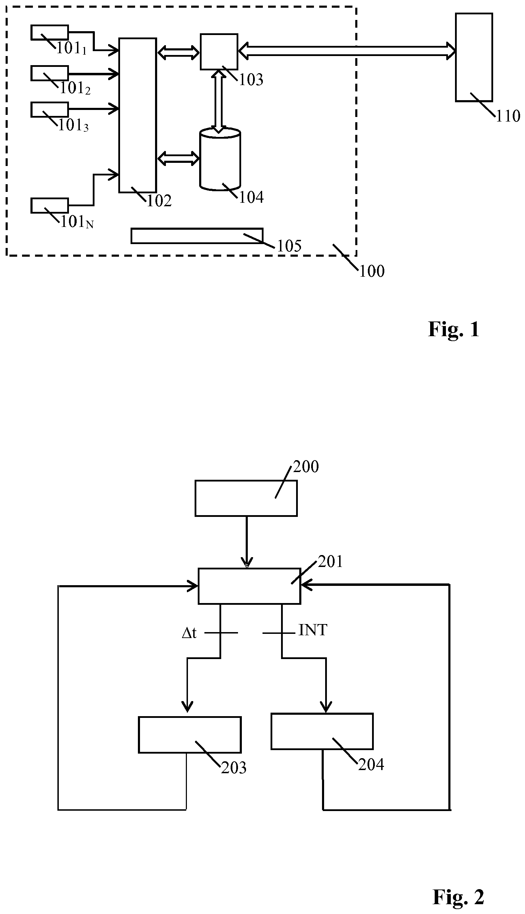

Un exemple d'architecture fonctionnelle d'un dispositif pour instrument chirurgical selon l'invention est illustré par la figure 1.

Les éléments fonctionnels représentés peuvent être logés dans un boîtier 100 qui peut être localisés à différents endroits de l'instrument, par exemple au niveau de la poignée. Il peut être envisagée que certains éléments soient localisés à d'autres endroits de l'instrument. Dans ce cas, il peut être possible de disposer plusieurs boîtiers distincts, chacun protégeant des éléments différents du dispositif.

Le boîtier 100 doit répondre aux requis des normes sanitaires en usage dans le domaine d'application.

Un instrument chirurgical est amené à subir des cycles de nettoyages automatiques et/ou manuels avec des produits détergents. Il peut être également amené à subir un traitement de stérilisation après chaque intervention. Le boîtier doit donc être à même de protéger les éléments propres à l'invention de ce traitement. Aussi, le boîtier 100 doit être hermétique et résistant à la chaleur et à la pression.

Plus particulièrement, selon les normes actuellement en vigueur, il doit être constitué d'un matériau résistant à une température d'environ 135°C degrés et à une pression d'environ 3 bars.

En outre, le boitier peut être prévu pour protéger les éléments qu'il contient des chocs, notamment des chocs liés à son fonctionnement normal mais aussi certains chocs accidentels tels que ceux résultant d'une chute d'un plan de travail ou d'un lieu de stockage (donc typiquement d'une hauteur d'environ 1 à 2 mètres.)

Il en résulte que des mises en œuvre possibles du boitier sont des boitiers ne permettant pas d'être ouverts. Une telle mise en oeuvre possible est un surmoulage en matériau polymère, comme par exemple le silicone, ou en matériau thermoplastique, comme par exemple le RADEL@ R, qui répondent effectivement à toutes les contraintes exposées ci-dessus.

Dans le cadre de telles mises en oeuvre, il n'est donc plus possible d'accéder à la partie électronique mettant en oeuvre l'invention.

Par conséquent, préférentiellement, celle-ci doit répondre à des contraintes de durée de vie n'abaissant pas celle de l'instrument chirurgical en lui-même. Les différents éléments doivent donc être choisis tels que leur durée de vie estimée (ou MTBF pour *« Mean Time Between Failure* ») doit au moins supérieure à celle souhaitée pour l'instrument chirurgical (qui peut correspondre à celle de l'instrument ne mettant pas en oeuvre l'invention).

Une autre contrainte est l'apport en énergie pour ces éléments qui doit donc provenir de l'intérieur du boitier lui-même, afin que l'ensemble des éléments qu'il contient puisse fonctionner de façon autonome. Le boitier 100 peut donc contenir une batterie 105, dimensionnée de sorte à permettre une alimentation énergétique suffisante pour permettre un fonctionnement des différents autres éléments contenus dans le boitier pendant la durée de vie souhaitée de l'instrument chirurgical et selon une utilisation estimée normale de l'instrument.

L'arrivée sur le marché de batteries rechargeables à substrats « solides » permettrait, dans un volume compatible d'un instrument chirurgical, de faire face aux conditions de température et de pression décrites précédemment. Ceci, associé à des processus de récupération d'énergie autonomes issus par exemple des montées en températures (lors de stérilisations), des communications RFID ou des mouvements ou vibrations de l'instrument, pourraient avantageusement allonger la durée de vie du module électronique de l'instrument.

Cette même contrainte influe sur les mécanismes mis en oeuvre par les capteurs et le circuit de contrôle et de traitement qui seront expliqués plus loin, afin de minimiser la consommation énergétique des différents éléments du boîtier.

Le boitier 100 comporte des capteurs 101₁, 101₂, 101₃,... 101_{N}, dans lequel N est le nombre de capteurs. Ce nombre peut être éventuellement égal à 1, signifiant que le boîtier ne comporte qu'un unique capteur.

L'invention est susceptible de s'adapter à tout type de capteurs et donc ceux-ci peuvent être de natures variées.

Il peut notamment s'agir de capteurs de type mécanique. Un exemple de capteur mécanique est un contacteur bilame, ou interrupteur thermique bilame, qui en fonction de la température ouvre ou ferme un circuit électrique permettant ainsi de détecteur le passage de la température ambiante au dessus d'un certain seuil.

Il peut également s'agir de capteurs de type électronique. Un exemple de capteur électronique est un accéléromètre, qui peut être un accéléromètre 3-axes.

Ces capteurs sont préférentiellement choisis avec pour critère important une faible consommation énergétique.

Les capteurs sont connectés électroniquement et logiquement à un circuit 102 de contrôle et de traitement. Ce circuit permet de contrôler les capteurs et de traiter les signaux émis par ceux-ci. En réponse à ces signaux, il peut fournir des informations qui sont alors stockées dans une mémoire 104 de type EEPROM.

Le but du circuit 102 est de déterminer les informations relatives à un certain nombre de mesures souhaités : ces mesures peuvent par exemple concerner le nombre de fois que l'instrument a chuté, le nombre de fois qu'il a subit une stérilisation, le nombre de fois qu'il a été utilisé, etc.

Typiquement, le circuit 102 détecte donc des événements (chute, choc, etc.), grâce aux capteurs, et mets à jour des compteurs correspondants aux mesures souhaitées. Ces mesures souhaitées peuvent être déterminées à la fois par construction (les capteurs installés dans le boîtier 100), mais aussi, partiellement par programmation.

Les informations ainsi déterminées par le circuit 102 sont ensuite accessibles depuis un dispositif extérieur 110 au moyen d'une interface radio 103. Cette interface radio peut typiquement être un émetteur passif tel qu'une étiquette RFID rendant accessible le contenu de la mémoire 104.

L'accès à ces données par le dispositif extérieur 110 étant avantageusement alimenté par celui-ci, par l'intermédiaire de la technologie RFID, cet accès restera possible en cas de décharge ou de défaillance de la source embarquée.

Le contrôle et le traitement déployés par le circuit 102 selon une mise en oeuvre de l'invention sont schématisés sur la figure 2.

Dans une première étape 200, le circuit 102 contrôle les capteurs en programmant ceux qui peuvent l'être.

Les capteurs concernés sont les capteurs électroniques, tels que les accéléromètres. Ceux-ci peuvent être paramétrés de différentes façons, en fonction du type de signal recherché et donc du type d'événements que le circuit cherche à mesurer. La façon de les programmer peut également dépendre de la capacité de programmation des capteurs.

Plusieurs situations peuvent donc être à envisager.

Dans un premier cas, le capteur peut être programmé afin de détecter un événement « chute ». Plus précisément, il peut s'agir de programmer un accéléromètre pour émettre une interruption matérielle destinée au circuit 102 lorsqu'il détecte une accélération correspondant à une chute libre pendant au moins un temps prédéfini (qui peut être réglable).

Dans un second cas, le capteur ne peut pas être programmé afin de déterminer un type d'événement. Il peut par exemple s'agir de la détection de chocs, car il peut être important, voire déterminant, de comprendre ce type d'événement avec des données contextuelles.

Aussi, le capteur peut être programmé pour repérer grossièrement l'événement à mesurer. Il s'agit de mettre en place une sur-détection en définissant un profil d'événement assez général. Typiquement pour l'exemple du choc, il s'agit de fournir un seuil bas d'accélération qui pourrait correspondre à un choc.

Dans le même temps, le capteur est alors programmé pour mémoriser ses valeurs pendant un temps et selon un échantillonnage prédéfinis.

De la même façon que précédemment, en cas de détection de l'événement, le capteur émettra une interruption au circuit 102, mais cette fois-ci, comme nous le verrons plus loin, le circuit mettra en oeuvre un traitement plus avancé (qu'il n'est pas possible de déployer sur un simple capteur) afin de filtrer les événements et éviter la sur-détection.

Une fois la programmation des capteurs effectuée, le circuit 102 met en oeuvre une étape 201 de mise en veille. Cette mise en veille permet de diminuer considérablement la consommation en énergie globale des éléments contenus dans le boitier 100, dans la mesure où le circuit 102 est le circuit le plus coûteux énergétiquement.

Cette mise en veille peut être autoprogrammée pour permettre une sortie de veille dans deux situations indépendantes : l'écoulement d'un lapse de temps Δt prédéfini, et la réception d'une interruption provenant d'un quelconque des capteurs 101₁, 101₂, 101₃,... 101_{N}.

La sortie périodique et le déploiement de l'étape 203 correspondante sont optionnels et peuvent correspondre à une double vérification :
- La vérification de l'état de la source d'énergie (ou batterie) 105. Si celle-ci descend au dessous d'un certain seuil, une information peut être stockée dans la mémoire 104 afin de l'indiquer aux utilisateurs via le dispositif externe 110.
- La vérification de la consistance de la mémoire 104 avec la mémoire interne du circuit 102. Il peut en effet survenir un problème de communication sur le bus reliant le circuit 102 et la mémoire 104. Auquel cas, le circuit 102 va itérer plusieurs transmission sur le bus, mais, afin de minimiser la consommation en énergie, au bout d'un nombre prédéfini d'itérations, il peut abandonner mais mets à jour les informations dans sa mémoire interne. Lors d'un prochain réveil, il peut ainsi déterminer une inconsistance et tenter de nouvelles écritures dans la mémoire 104.

A la suite de ces deux vérifications, le circuit 102 peut se remettre en état de veille. Ainsi, donc, selon cette mise en oeuvre, le circuit sort périodiquement et très temporairement de l'état de veille. Le traitement correspondant aux deux vérifications est suffisamment court pour, si la périodicité Δt n'est pas trop courte, ne pas impacter significativement la durée de vie de la batterie 105.

Indépendamment, le circuit 102 peut sortir de l'état de la veille à la réception d'une interruption provenant d'un des capteurs. Comme il a été vu précédemment, il peut s'agir d'une interruption liée à un changement d'état d'un capteur de type mécanique, ou à la détection de conditions déclenchantes par un capteur de type électronique.

Le circuit 102 met alors en oeuvre une étape 204 de traitement

Ce traitement peut dépendre de la nature du capteur ayant transmis l'interruption et de la nature de la mesure considérée.

D'une façon générale, le traitement consiste à incrémenter un compteur stocké dans la mémoire 104. De la sorte, la mémoire 104 contient l'historique de l'instrument chirurgical, par exemple
- le nombre de fois qu'il est tombé, déterminé par le nombre de chutes libres détectés par un accéléromètre ;
- le nombre de fois qu'il a été stérilisé, déterminé par le nombre de détections d'une température au dessus d'un seuil prédéterminé et fonction des températures usuelles de stérilisation ;
- le nombre de fois qu'il a été utilisé, déterminé par exemple par un nombre d'actionnements d'un capteur approprié, ou le nombre de détections par un accéléromètre paramétré et positionné de façon approprié ;
etc.

Au préalable, un post-traitement peut être appliqué sur le signal transmis par un capteur. Comme nous l'avons vu précédemment, des capteurs peuvent ne pas être programmés de sorte à détecter toutes sortes d'événements. Dans ce cas, ceux-ci sont programmés afin d'éviter de rater un événement, mais au détriment d'un taux de sur-détection. Aussi, dans l'étape 204, le circuit 102 met en oeuvre un traitement plus fin permettant de diminuer le taux de sur-détection.

Notamment, il peut recueillir un ensemble d'échantillons capturés par le capteur autour de l'événement détecté et le comparer à un ensemble de signatures ou gabarits afin de déterminer si l'interruption reçue correspond effectivement à un événement réel ou bien s'il s'agit d'une sur-détection devant être écarté.

Une fois l'interruption traitée, le circuit peut alors se remettre en état de veille (boucle vers l'étape 201).

En outre, le boîtier peut contenir une interface radio 103, notamment une étiquette RFID. Cette étiquette RFID permet de rendre accessible en lecture le contenu de la mémoire 104 à des dispositifs extérieurs 110 disposant de lecteurs RFID (pour « *Radio-Frequency Identification* » en langue anglaise). D'autres mécanismes radio sont également envisageables (Bluetooth, etc.)

Ce dispositif extérieur peut être un terminal mobile de télécommunication de type « smart phone », une tablette numérique ou tout autre équipement de traitement de données (ordinateur fixe ou portable, etc.).

Ce dispositif extérieur 110 dispose d'une interface homme-machine (embarquée ou déportée) permettant l'accès à un utilisateur d'accéder aux informations stockées dans la mémoire 104. Ainsi, le personnel hospitalier peut accéder à des informations lui permettant de juger au mieux de l'opportunité de procéder au recalibrage ou au remplacement d'un instrument chirurgical donné. Il en résulte une gestion plus fine d'un parc d'instruments, et une réduction des coûts et des risques.

Les distributeurs et les fabricants peuvent en outre accéder à ces informations et peuvent ainsi disposer de métriques sur l'utilisation de leurs instruments « sur le terrain ». Ils peuvent tirer profit de ces données pour adapter leurs offres commerciales mais aussi éventuellement la conception de leurs instruments.

L'interface radio 103 peut être également prévue pour recevoir des informations du dispositif extérieur 110 et pour les stocker dans la mémoire 104. Ces informations peuvent être propres au service et/ou à l'établissement utilisant l'instrument chirurgical, et pourront ainsi permettre de suivre la vie de l'instrument. Bien évidemment, uniquement une partie de la mémoire 104 peut être rendu accessible en écriture, afin d'éviter des manipulations erronées ou frauduleuses des informations issues des capteurs.

Il est possible de protéger l'accès aux informations stockées dans la mémoire 104 à travers un mécanisme de sécurisation. Il est également possible de compartimenter tout ou partie des informations afin que différents acteurs (personnel hospitalier, distributeur, fabricant...) n'est accès, en lecture et/ou en écriture, qu'à une partie prédéfinie de ces informations.

Le mécanisme de sécurisation peut être un couple nom d'utilisateur / mot de passe qui est stocké dans l'instrument (par exemple dans une zone système sécurisé de la mémoire 104) et transmis par ailleurs aux différents acteurs (courrier électronique, courrier postal, etc.)

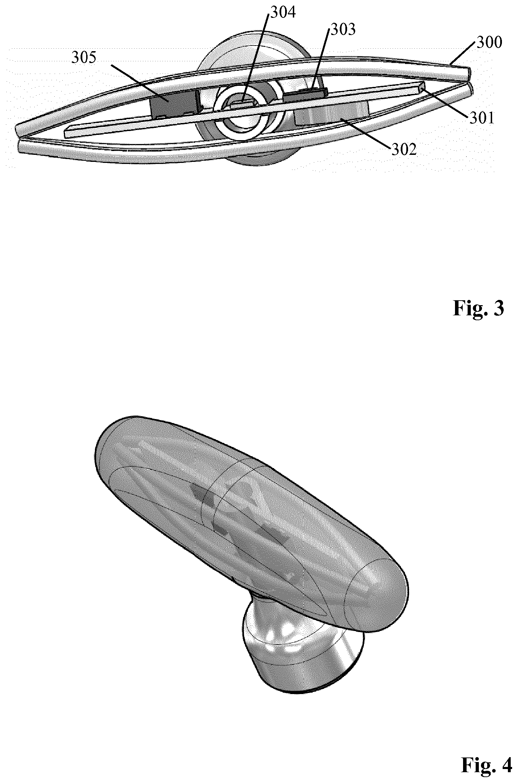

Les figures 3 et 4 schématisent un instrument chirurgical particulier mettant en oeuvre l'invention. Cet instrument est un outil pour la chirurgie du rachis. Sur les figures 3 et 4 ne sont représentés que la poignée de cet instrument, dans laquelle sont logés les différents éléments de l'invention.

Cette poignée comprend un corps 300 assurant le maintient des éléments et la rigidité de la poignée. Ce corps forme un logement dans lequel se situe une assise 302 recevant les différents éléments 302, 303, 304, 305. Le corps 300 possède donc pour fonction supplémentaire de protéger les éléments des chocs (chutes) et de la préhension de la poignée par le praticien.

Sur l'assise sont disposés une batterie 302, le circuit 303 pour le contrôle et le traitement des capteurs 304, 305.

Un premier capteur 305 est un contacteur bilame visant à compter le nombre de stérilisation dont l'instrument fait l'objet.

Le second capteur 304 est un accéléromètre 3 axes. Il permet de compter le nombre de chutes de l'instrument, mais aussi le nombre de déclenchements dans le cadre d'un limiteur de couple, par la détection d'une signature donnée.

Ce type d'instruments pour la chirurgie du rachis étant à la fois critique en terme de dangerosité pour le patient opéré) et sensible aux déviations de sa calibration, la connaissance des informations mesurées grâce à l'invention permet donc de procéder à son recalibrage ou à son remplacement de façon optimale.

La figure 4 représente une autre vue de la même poignée, dans laquelle les différents éléments sont logés dans un boitier qui est un surmoulage, par exemple en silicone. Ce surmoulage est représenté sur la figure de façon semi-transparente pour la compréhension de ce mode de réalisation de l'invention.

Selon un mode de réalisation de l'invention, le dispositif peut être prévu pour s'associer à une pluralité d'instruments chirurgicaux. Par exemple, le dispositif peut être localisé sur une caisse dédiée au transport et à la stérilisation de kits d'instruments. Ce dispositif comporte alors notamment un capteur de température (bilame...) pour compter le nombre de stérilisation.

Ainsi, plusieurs instruments chirurgicaux munis du dispositif 100 et constituant un kit complet d'ancillaires pourront être contenus dans une caisse (métallique ou d'un autre matériau) dédiée au transport et/ou à la stérilisation de kits d'instruments, elle-même équipée du dispositif 100.

Grâce à un dispositif extérieur 101 muni d'une antenne RFID à longue distance de communication, les données enregistrées dans l'ensemble des dispositifs (100) précédemment cités pourront être acquises sensiblement simultanément par ce dispositif extérieur unique. La caisse peut être fermée et complète, et l'acquisition des données peut se faire à partir d'une distance supérieure à 50 cm.

L'ensemble des données récupérées, ainsi associées au kit complet, permettant d'en vérifier la composition (chaque instrument est bien dans la caisse où il a été originalement placé), la traçabilité (le kit sort du bloc opératoire, de la zone de nettoyage/stérilisation, des zones de stockage du fournisseur) et la conformité (les instruments contenus sont un à un conformes), seront alors affichées sur un dispositif extérieur 101 à l'accès sécurisé et transmises par celui-ci, via internet, à une base de données consultable d'une interface graphique, elle aussi sécurisée.

Le dispositif extérieur 110 peut disposer d'une interface homme-machine permettant l'affichage et/ou peut transmettre ces informations, via internet, à une base de données consultable à partir d'une interface homme-machine déportée.

De façon préférentielle, les transmissions, l'interface homme-machine, le dispositif extérieur 110 sont sécurisés.

Selon un autre mode de réalisation, des modules autonomes peuvent être positionnés à des endroits précis lors de l'opération chirurgical et capter des informations fournies par un ou plusieurs dispositifs associés à des instruments chirurgicaux.

Plusieurs variantes sont donc possibles en sus de celles décrites en connexion avec les figures, pour mettre en oeuvre un dispositif pour un instrument chirurgical.

Ainsi, la présente invention n'est pas limitée aux exemples et au mode de réalisation décrits et représentés, mais elle est susceptible de nombreuses variantes accessibles à l'homme de l'art.

## Revendications

1. Instrument chirurgical avec un dispositif localisé sur l'instrument, le dispositif comportant au moins un capteur (101₁, 101₂, 101₁,...101_{N}), un circuit (102) de contrôle et de traitement des signaux issus dudit au moins un capteur, une mémoire (104) pour stocker des informations issues dudit circuit, et une interface radio (103) pour transmettre lesdites informations à un dispositif extérieur (110), ledit au moins un capteur, ledit circuit, ladite mémoire et ladite interface radio étant alimentés par une source d'énergie (105), ledit circuit, ladite mémoire, ladite interface radio et ladite source d'énergie étant logés dans un ou plusieurs boîtiers (100) hermétiques et résistants à la chaleur et à la pression, afin de permettre un fonctionnement autonome dudit dispositif.

2. Dispositif selon la revendication 1, dans lequel ledit boitier est un surmoulage en matériau polymère ou thermoplastique.

3. Dispositif selon l'une des revendications précédentes, dans lequel ladite interface radio (103) est une étiquette RFID.

4. Dispositif selon l'une des revendications précédentes, dans lequel ledit au moins un capteur comprend un accéléromètre et un contacteur bilame.

5. Dispositif selon l'une des revendications précédentes, dans lequel ladite interface radio (103) est prévue pour recevoir des secondes informations dudit dispositif extérieur (110) pour les stocker dans ladite mémoire (104).

6. Dispositif selon l'une des revendications précédentes, dans lequel les capteurs sont prévus pour transmettre une interruption audit circuit (102), lors de la survenance d'un événement prédéfinis, et dans lequel ledit circuit est adapté pour se maintenir dans un état de veille jusqu'à la réception de ladite interruption et pour alors traiter les signaux issus du capteur correspondant à ladite interruption, puis se remettre en état de veille.

7. Dispositif selon la revendication précédente, dans lequel ledit circuit (102) est prévu pour sortir périodiquement et temporairement de l'état de veille afin de vérifier la consistance du contenu entre ladite mémoire (104) et sa mémoire interne et l'état de ladite source d'énergie.

8. Dispositif selon l'une des revendications précédentes, dans lequel ledit boîtier est constitué d'un matériau résistant à une température d'environ 135°C degrés et à une pression d'environ 3 bars.

9. Système comportant un dispositif (100) selon l'une des revendications précédentes et un dispositif extérieur (110) prévu pour permettre la visualisation des informations reçues de ladite interface radio (103).

10. Système selon la revendication précédente comportant un ensemble d'instruments chirurgicaux munis de premiers dispositifs selon l'une des revendications 1 à 8 et une caisse de transport muni d'un second dispositif (100) comportant au moins un capteur (101₁, 101₂, l01₁,...101_{N}), un circuit (102) de contrôle et de traitement des signaux issus dudit au moins un capteur, une mémoire (104) pour stocker des informations issues dudit circuit, et une interface radio (103) pour transmettre lesdites informations à un dispositif extérieur (110), ledit au moins un capteur, ledit circuit, ladite mémoire et ladite interface radio étant alimentés par une source d'énergie (105), ledit circuit, ladite mémoire, ladite interface radio et ladite source d'énergie étant logés dans un ou plusieurs boîtiers (100) hermétiques et résistants à la chaleur et à la pression, afin de permettre un fonctionnement autonome dudit second dispositif.

11. Procédé de gestion de l'instrument chirurgical avec le dispositif, selon la revendication 1, comportant une étape préalable de stockage d'informations dans la mémoire du dispositif associé audit instrument, issues d'au moins un des capteurs intégrés dans ledit dispositif, ladite mémoire étant alimentée par la source d'énergie, ladite mémoire et ladite source étant logées dans au moins un boîtier (100) hermétique et résistant à la chaleur et à la pression afin de permettre un fonctionnement autonome dudit dispositif, puis une étape ultérieure (201) de transmission desdites informations stockées dans la mémoire dudit dispositif vers un dispositif extérieur et d'affichage desdites informations sur une interface homme-machine de ce dispositif extérieur.

12. Procédé de gestion d'un ensemble d'instruments chirurgicaux contenus dans une caisse de transport, ledit procédé comportant l'utilisation du procédé selon la revendication précédente pour chacun des instruments dudit ensemble afin que lesdites informations soient transmises à un dispositif extérieur (110) unique, ainsi qu'une étape préalable de stockage d'informations dans une mémoire d'un dispositif associé à ladite caisse, issues d'au moins un capteur intégré dans ledit dispositif, ladite mémoire étant alimentée par une source d'énergie, ladite mémoire et ladite source étant logées dans au moins un boitier (100) hermétique et résistant à la chaleur et à la pression afin de permettre un fonctionnement autonome dudit dispositif, puis une étape ultérieure (201) de transmission desdites informations stockées dans la mémoire dudit dispositif vers ledit dispositif extérieur, ledit dispositif extérieur permettant l'affichage des informations reçues de ladite caisse et desdits instruments chirurgicaux sur une interface homme-machine.

## Patentansprüche

1. Chirurgisches Instrument mit einer Vorrichtung, die sich auf dem Instrument befindet, die Vorrichtung umfassend mindestens einen Sensor (101₁, 101₂, 101₁,...101_{N}), eine Schaltung (102) zum Steuern und Verarbeiten von Signalen, die von dem mindestens einen Sensor ausgegeben werden, einen Speicher (104) zum Speichern von Informationen, die von der Schaltung ausgegeben werden, und eine Funkschnittstelle (103) zum Übertragen der Informationen an eine externe Vorrichtung (110), wobei der mindestens eine Sensor, die Schaltung, der Speicher und die Funkschnittstelle von einer Energiequelle (105) gespeist werden, wobei die Schaltung, der Speicher, die Funkschnittstelle und die Energiequelle in einem oder mehreren hermetischen, hitze- und druckbeständigen Gehäusen (100) untergebracht sind, um einen autonomen Betrieb der Vorrichtung zu ermöglichen.

2. Vorrichtung nach Anspruch 1, wobei das Gehäuse eine Umspritzung aus einem polymeren oder thermoplastischen Material ist.

3. Vorrichtung nach einem der vorherigen Ansprüche, wobei die Funkschnittstelle (103) ein RFID-Tag ist.

4. Vorrichtung nach einem der vorherigen Ansprüche, wobei der mindestens eine Sensor einen Beschleunigungsmesser und ein Bimetall-Schaltschütz umfasst.

5. Vorrichtung nach einem der vorherigen Ansprüche, wobei die Funkschnittstelle (103) bereitgestellt ist, um zweite Informationen von der externen Vorrichtung (110) zu empfangen, um sie in dem Speicher (104) zu speichern.

6. Vorrichtung nach einem der vorherigen Ansprüche, wobei die Sensoren bereitgestellt sind, um beim Auftreten eines vordefinierten Ereignisses eine Unterbrechung an die Schaltung (102) zu übertragen, und wobei die Schaltung angepasst ist, um bis zum Empfangen der Unterbrechung in einem Bereitschaftszustand zu bleiben und dann die von dem Sensor ausgegebenen Signale zu verarbeiten, die der Unterbrechung entsprechen, und dann wieder in den Bereitschaftszustand zu wechseln.

7. Vorrichtung nach dem vorherigen Anspruch, wobei die Schaltung (102) bereitgestellt ist, um den Bereitschaftszustand periodisch und vorübergehend zu verlassen, um die Konsistenz des Inhalts zwischen dem Speicher (104) und seinem internen Speicher und den Zustand der Energiequelle zu überprüfen.

8. Vorrichtung nach einem der vorherigen Ansprüche, wobei das Gehäuse aus einem Material besteht, das einer Temperatur von etwa 135 °C Grad und einem Druck von etwa 3 bar standhält.

9. System, umfassend eine Vorrichtung (100) nach einem der vorherigen Ansprüche und eine externe Vorrichtung (110), die bereitgestellt ist, um die Anzeige der von der Funkschnittstelle (103) empfangenen Informationen zu ermöglichen.

10. System nach dem vorherigen Anspruch, umfassend eine Einheit chirurgischer Instrumente, die mit ersten Vorrichtungen gemäß einem der Ansprüche 1 bis 8 versehen sind, und eine Transportkiste, die mit einer zweiten Vorrichtung (100) versehen ist, umfassend mindestens einen Sensor (101₁, 101₂, 101₁,... 101_{N}), eine Schaltung (102) zum Steuern und Verarbeiten von Signalen, die von dem mindestens einen Sensor ausgegeben werden, einen Speicher (104) zum Speichern von Informationen, die von der Schaltung ausgegeben werden, und eine Funkschnittstelle (103) zum Übertragen der Informationen an eine externe Vorrichtung (110), wobei der mindestens eine Sensor, die Schaltung, der Speicher und die Funkschnittstelle von einer Energiequelle (105) gespeist werden, wobei die Schaltung, der Speicher, die Funkschnittstelle und die Energiequelle in einem oder mehreren hermetischen, hitze- und druckbeständigen Gehäusen (100) untergebracht sind, um einen autonomen Betrieb der zweiten Vorrichtung zu ermöglichen.

11. Verfahren zur Verwaltung des chirurgischen Instruments mit der Vorrichtung nach Anspruch 1, umfassend einen vorherigen Schritt eines Speicherns von Informationen in dem Speicher der mit dem Instrument assoziierten Vorrichtung, die von mindestens einem der in die Vorrichtung integrierten Sensoren ausgegeben werden, wobei der Speicher von der Energiequelle gespeist wird, wobei der Speicher und die Quelle in mindestens einem hermetischen und hitze- und druckbeständigen Gehäuse (100) untergebracht sind, um einen autonomen Betrieb der Vorrichtung zu ermöglichen, sowie einen späteren Schritt (201) zum Übertragen der im Speicher der Vorrichtung gespeicherten Informationen zu einer externen Vorrichtung und zum Anzeigen der Informationen auf einer Mensch-Maschine-Schnittstelle dieser externen Vorrichtung.

12. Verfahren zur Verwaltung einer Einheit von chirurgischen Instrumenten, die in einer Transportkiste enthalten sind, das Verfahren umfassend die Verwendung des Verfahrens nach dem vorherigen Anspruch für jedes der Instrumente der Einheit, damit die Informationen an eine einzige externe Vorrichtung (110) übertragen werden, sowie einen vorherigen Schritt eines Speicherns von Informationen in einem Speicher einer Vorrichtung, die mit der Kiste assoziiert ist, die von mindestens einem in die Vorrichtung integrierten Sensor ausgegeben werden, wobei der Speicher von einer Energiequelle gespeist wird, wobei der Speicher und die Quelle in mindestens einem hermetischen und hitze- und druckbeständigen Gehäuse (100) untergebracht sind, um einen autonomen Betrieb der Vorrichtung zu ermöglichen, sowie einen späteren Schritt (201) zum Übertragen der in dem Speicher der Vorrichtung gespeicherten Informationen an die externe Vorrichtung, wobei die externe Vorrichtung die Anzeige der empfangenen Informationen der Kiste und der chirurgischen Instrumente an einer Mensch-Maschine-Schnittstelle ermöglicht.

## Claims

1. Surgical instrument with a device located on the instrument, the said device comprising at least one sensor (101₁, 101₂, 101₁,...101N), a circuit (102) for controlling and processing signals originating from the said at least one sensor, a memory module (104) for storing information originating from the said circuit, and a radio interface (103) for transmitting the said information to an external device (110), the said at least one sensor, the said circuit, the said memory module and the said radio interface being powered by a power source (105), the said circuit, the said memory module, the said radio interface and the said power source being housed in one or more hermetically sealed heat-resistant and pressure-resistant casings (100), in order to allow autonomous operation of the said device.

2. Device according to claim 1, wherein the said casing is an overmoulding made from a polymeric or thermoplastic material.

3. Device according to one of the preceding claims, wherein the said radio interface (103) is an RFID tag.

4. Device according to one of the preceding claims, wherein the said at least one sensor comprises an accelerometer and a bimetallic switch.

5. Device according to one of the preceding claims, wherein the said radio interface (103) is configured to receive second information from the said external device (110) in order to store the said second information in the said memory module (104).

6. Device according to one of the preceding claims, wherein the sensors are configured to transmit an interruption to the said circuit (102) upon the occurrence of a predefined event, and wherein the said circuit is adapted to remain in a standby state until the said interruption is received and then to process the signals from the sensor corresponding to the said interruption, then return to a standby state.

7. Device according to the preceding claim, wherein the said circuit (102) is configured to periodically and temporarily leave the standby state in order to check the consistency of the content between the said memory module (104) and its internal memory and the state of the said power source.

8. Device according to one of the preceding claims, wherein the said casing is made from a material resistant to a temperature of approximately 135° C and a pressure of approximately 3 bars.

9. System comprising a device (100) according to one of the preceding claims and an external device (110) configured to allow the display of information received from the said radio interface (103).

10. System according to the preceding claim, comprising a set of surgical instruments containing first devices according to one of claims 1 to 8 and a transport case containing a second device (100) comprising at least one sensor (101₁, 101₂, 101₁,... 101_{N}), a circuit (102) for controlling and processing signals originating from the said at least one sensor, a memory module (104) for storing information originating from the said circuit, and a radio interface (103) for transmitting the said information to an external device (110), the said at least one sensor, the said circuit, the said memory module and the said radio interface being powered by a power source (105), the said circuit, the said memory module, the said radio interface and the said power source being housed in one or more hermetically sealed heat-resistant and pressure-resistant casings (100), in order to allow autonomous operation of the said second device.

11. Method for managing the surgical instrument with the device, according to claim 1, comprising a preliminary step of storing information in the memory module of the device associated with the said instrument, originating from at least one of the sensors integrated into the said device, the said memory module being powered by the power source, the said memory module and the said source being housed in at least one hermetically sealed heat-resistant and pressure-resistant casing (100) in order to allow autonomous operation of the said device, then a subsequent step (201) of transmitting the said information stored in the memory module of the said device to an external device and of displaying the said information on a human-machine interface of the said external device.

12. Method for managing a set of surgical instruments contained in a transport case, the said method comprising the use of the method according to the preceding claim for each of the instruments of the said set so that the said information is transmitted to a single external device (110), as well as a preliminary step of storing information in a memory module of a device associated with the said case, originating from at least one sensor integrated into the said device, the said memory module being powered by a power source, the said memory module and the said source being housed in at least one hermetically sealed heat-resistant and pressure-resistant casing (100) in order to allow autonomous operation of the said device, then a subsequent step (201) of transmitting the said information stored in the memory module of the said device to the said external device, the said external device allowing the display of the information received from the said case and from the said surgical instruments on a human-machine interface.
